**Europäisches Patentamt**

**European Patent Office**

**Office européen des brevets**

(19)

(11) Publication number : **0 469 049 B1**

# (12) EUROPEAN PATENT SPECIFICATION

(45) Date of publication of patent specification :
30.09.92 Bulletin 92/40

(51) Int. Cl.⁵ : **C11C 3/10,** C12P 7/64

(21) Application number : **90907177.1**

(22) Date of filing : **18.04.90**

(86) International application number :
**PCT/DK90/00101**

(87) International publication number :
**WO 90/12858 01.11.90 Gazette 90/25**

(54) PROCESS FOR PREPARATION OF TRIGLYCERIDE AND TRIGLYCERIDE COMPOSITION.

(30) Priority : **19.04.89 DK 1906/89**

(43) Date of publication of application :
**05.02.92 Bulletin 92/06**

(45) Publication of the grant of the patent :
**30.09.92 Bulletin 92/40**

(84) Designated Contracting States :
**AT BE CH DE DK ES FR GB IT LI LU NL SE**

(56) References cited :
**EP-A- 0 305 901**
**GB-A- 2 147 004**
**GB-A- 2 159 527**
**Chemical Abstracts, vol. 103, no. 19, 11
November 1985, (Columbus, Ohio, US), Mac-
rae, A.R.: "Microbial lipases as catalystsfor
the interesterification of oils and fats", p. 581,
abstract 159056q**
**Patent Abstracts of Japan, vol. 11, no. 300,
C449, abstract of JP 62-91188, 25.04.1987, Nis-
shin Oil Mills Ltd.**

(73) Proprietor : **NOVO NORDISK A/S**
**Novo Allé**
**DK-2880 Bagsvaerd (DK)**
Proprietor : **LYSI HF**
**Grandavegur 42**
**IS-121 Reykjavik (IS)**

(72) Inventor : **HARALDSSON, Gudmundur, G.**
**Skogaras 11**
**IS-110 Reykjavik (IS)**
Inventor : **SVANHOLM, Hanne**
**Gladsaxevej 80**
**DK-2860 Soeborg (DK)**
Inventor : **HANSEN, Tomas, Tage**
**Tonedraget 5**
**DK-3450 Alleroed (DK)**
Inventor : **HJALTASON, Baldur**
**Nordurstigur 3**
**IS-101 Reykjavik (IS)**

(74) Representative : **Knudsen, Sten Lottrup et al
c/o Novo Nordisk A/S, Patent Department,
Novo Allé
DK-2880 Bagsvaerd (DK)**

## Description

## TECHNICAL FIELD

The invention relates to a process for the preparation of a triglyceride having at least one long-chain fatty acid (e.g. a polyunsaturated acid) in the molecule. It also relates to a triglyceride composition with a high content of polyunsaturated fatty acid in the triglyceride molecules.

## BACKGROUND ART

It is known that triglycerides of poly-unsaturated fatty acid (PUFA) , such as eicosapentaenoic acid (EPA) and docosahexaenoic acid (DHA), have beneficial medical effects, and within the last decade much attention has been directed to methods of producing triglyceride compositions with a high content of these acids, and particularly a high content of triglycerides with three such acids in the molecule.

PUFA in the form of free fatty acid or lower alkyl (e.g. methyl or ethyl) ester is available in high purity and have been used to prepare triglycerides with high PUFA content.

Thus, JP-A 61-43143 (Nisshin Flour Mill et al.) and EP 300,844 (R.F. Azar et al.) describe chemical interesterification of lower alkyl PUFA ester with triacetin or tributyrin. Sodium methylate was used as catalyst, and vacuum was used to remove lower alkyl acetate or butyrate formed in the reaction. The former describes production of triglyceride with 90% PUFA content.

JP-A 61-246146 (Nissui Seiyaku) describes halogenation of PUFA free acid, followed by reaction of PUFA acyl chloride with glycerine. The first step was carried out with oxalyl chloride at 65-90°C for 4 hours, and the second step under reflux for several hours in chloroform in the presence of quinoline or pyridine.

The above-mentioned processes use highly reactive chemicals that require special precautions in handling, these reactive chemicals react with part of the labile PUFA acyl groups, and the resulting reaction mixture in each case requires complex purification.

JP-A 62-91188 (Nisshin Oil) describes lipase-catalyzed production of PUFA glycerides from glycerol and PUFA free acid or ethyl ester, using positionally specific lipase in native form or immobilized on a weakly basic anion exchange resin. It is stated that addition of water is necessary. After reaction and removal of unreacted fatty acid, the glyceride mixture contained at most 86% triglyceride together with at least 14% diglyceride + monoglyceride. The product contained at most 85% polyunsaturated fatty acids together with at least 15% of other fatty acids.

It is an object of the invention to provide a simple process, avoiding the use of aggressive chemicals, to produce triglycerides of long-chain fatty acids with a low content of mono- and diglycerides. It is also an object to provide triglyceride compositions with high PUFA content.

## STATEMENT OF THE INVENTION

According to the invention, the objects are met by lipase-catalyzed interesterification of a long-chain free acid or lower alkyl ester thereof with a triglyceride of a short-chain acid. Short-chain free acid or a lower alkyl ester thereof is formed during the reaction, and utilizing its relatively high volatility is removed simultaneously by evaporation.

Accordingly, the invention provides a process for the preparation a triglyceride having at least one $C_{8+}$ long-chain fatty acid in the molecule, characterized by interesterification in the presence of a lipase of the corresponding long-chain free fatty acid or $C_1$-$C_4$ lower alkyl ester thereof with a triglyceride having one or more $C_2$-$C_6$ short-chain fatty acids in the molecule, and by evaporative removal during the reaction of short-chain free acid or lower alkyl ester thereof.

The invention also provides a triglyceride composition, characterized by at least 95 % by weight (preferably at least 98%) of the fatty acids in the triglyceride molecules being polyunsaturated $C_{18-22}$ acid.

## DETAILED DESCRIPTION OF THE INVENTION

### Long-chain fatty acid

The process of the invention is well suited for incorporation of $C_{12+}$ fatty acid since the reactant free acid or ester has very low volatility so the loss will be negligible. In particular, the process can be used for incorporating $C_{18}$-$C_{22}$ fatty acid which may be monounsaturated (e.g. oleic), diunsaturated (e.g. linoleic) or polyunsaturated (with three or more double bonds) such as linolenic acid, EPA or DHA. Due to the gentle conditions used in the process, by-product formation from these sensitive acids is largely avoided.

Thus, the process of the invention can be used to prepare triglyceride compositions with high content of such acid by using a reactant mixture wherein the free acid or lower alkyl ester comprises at least 90%, preferably at least 95% and most preferably at least 98%, of one or more such acids or esters thereof. In this way it is possible to prepare triglyceride with such acid in all three positions in good yield. In this connection, it has surprisingly been found that pure EPA or DHA is incorporated particularly fast.

Preferably, the long-chain fatty acid reactant is free fatty acid, methyl or ethyl ester; in this case the resulting short-chain acid or ester is very volatile and is easily removed.

The long-chain fatty acid or ester may be prepared by known methods, and some are commercially available in high purity, e.g. EPA and DHA as free acid and ethyl ester in 99% purity from Idemitsu Petro Chemical Co.,, Ltd., Japan.

Short-chain fatty acid

The short-chain acid present in the reactant triglyceride is preferably acetic or butyric acid, due to the volatility of the products. It is particularly advantageous to use triglyceride with three short-chain acids in the molecule, especially tributyrin or triacetin.

Lipase

Positionally specific (1,3-specific) or non-specific lipase may be used. If incorporation of long-chain acyl into all three positions of the triglyceride is desired, it is particularly advantageous to use non-specific lipase to achieve a fast reaction. The lipase must have sufficient stability at the temperature and reaction time to be used.

Examples of non-specific lipases are those derived from strains of Candida, especially C. antarctica lipase (WO 88/02775, incorporated herein by reference) , and lipase from C. rugosa (also known as C. cylindracea). It is particularly preferred to use a lipase preparation containing both lipase A and lipase B of C. antarctica described in said reference.

Examples of specific lipases are lipase derived from Humicola, especially H. lanuginosa (WO 89/06278) and recombinant Humicola lipase (EP 305,216) and Mucor lipase (EP 140,542).

Immobilized lipase is generally most effective for interesterification. Some immobilized lipases require a certain water content for activation. Depending on the reactants and reaction conditions, water may be given off by the evaporation. To compensate for this, it may be necessary to add water or immobilized lipase stepwise during reaction.

An example of a suitable immobilization method is adsorption of lipase on a particulate, macroporous adsorbent (i.e. non-ionic) resin of the acrylic type according to WO 89/02916.

An example of a lipase preparation that may be used is SP 382 from Novo Nordisk A/S (mixture of lipases A and B from C. antarctica, immobilized according to WO 89/02916).

Reaction conditions

A suitable amount of lipase is generally in the range 0.5 - 10 BIU/g (typically 1-5 BIU/g) of reactant mixture (BIU = Batch Interesterification Unit, see WO 89/06278) by use of immobilized lipase, or 50 - 500 LU/g of oil (LU = Lipase Unit, see WO 88/02775) by use of native (non-immobilized) lipase.

It is preferable to use the two reactants in the stoichiometric ratio or with a moderate excess (e.g. 0-50%, especially 0-20%) of the long-chain fatty acid reactant. And it is preferable to let the reaction continue until at least 70% (particularly more than 90, especially more than 95%, e.g. more than 98%) of the short-chain acid in the triglyceride molecule has been replaced with long-chain acid. This makes it possible to prepare triglyceride of long-chain fatty acid in good yield and purity.

It is generally not necessary to use a pH buffer or an organic solvent in the process.

A temperature of 40-80°C, especially 60-80°C, is generally suitable for the reaction and the evaporation. The reaction time will generally be below 72 hours.

Removal of volatile acid or ester

The removal by evaporation of volatile fatty acid (ester) may be done continuously from a stirred tank. Reactants may be added batch wise, semi-batch wise or continuously. The lipase may be immobilized and can be reused.

Alternatively, the reaction may occur in two or more steps, and evaporation can be done between the steps. Each process step can be made in a stirred tank, or immobilized lipase can be used continuously in a fixed bed.

The evaporation is most conveniently done under vacuum, e.g. below 200 Pa and especially below 20 Pa.

EXAMPLES

**EXAMPLES 1 - 4**

Preparation of triglycerides containing 20-85% EPA/DHA

Immobilized lipase derived from <u>Candida antarctica</u> (SP-382 from Novo-Nordisk A/S; activity approx. 30 BIU/g; 0.82-0.86g; 10 % moisture content) was added to a mixture of tributyrin (98-99% from Aldrich; 6.6 mmol) and fatty acid ethyl ester concentrate (19.8 mmol) of varying composition, as indicated below. The mixture was gently stirred at 65°C on a magnetic stirrer hot-plate under a continuous vacuum of 0.1 mm Hg. The volatile ethyl butyrate product was continuously condensed into a liquid nitrogen cooled trap, which could be separated and weighed regularly during the process by disconnecting the reaction by replacing the vacuum with dry nitrogen or argon atmosphere. After 72 hours the reaction was discontinued, hexane added and the enzyme separated off by filtration. After hexane removal <u>in vacuo</u> on rotary evaporator the crude reaction product was afforded. Hydrolysis was found to occur only to a minimum extent as indicated by titration, which demonstrated less than 1% free fatty acid content of the product and further established by Iatroscan studies. The product was freed from the remaining ethyl esters and purified by the aid of preparatory High Performance Liquid Chromatography (HPLC) eluting with 10% ether in pentane or hexane to afford 100% pure triglycerides as established by Iatroscan studies. Capillary Gas Liquid Chromatography (GLC) analysis in each case showed fatty acid composition almost identical to the original ethyl esters. The ethylbutyrate nature of the trapped component was established by Nuclear Magnetic Resonance (NMR). There was little or no sign of water coming over.

The following equation was used to calculate the % incorporation of fatty acids into tributyrin from the weight measurements:

$$\%\text{incorporation} = 0.867 \cdot [\mathbf{Wt}_{\text{ethyl bytyrate}} / \mathbf{Wt}_{\text{tributyrin}}] \cdot 100\%$$

This was deduced from the following equation:

$$\%\text{incorp.} = 1/3 \cdot [\text{\# of eq. of entrapped ethyl bytyrate} / \text{\# of eq. of tributyrin used}] \cdot 100\%$$
$$= 1/3 \cdot [\mathbf{Wt}_{\text{ethyl butyrate}} / \mathbf{M.wt}_{\text{ethyl butyrate}}] / [\mathbf{Wt}_{\text{tributyrin}} / \mathbf{M.wt}_{\text{tributyrin}}] \cdot 100\%$$
$$= 1/3 \cdot [\mathbf{Wt}_{\text{ethyl bytyrate}} / \mathbf{Wt}_{\text{tribytyrin}}] \cdot [\mathbf{M.wt}_{\text{tributyrin}} / \mathbf{M.wt}_{\text{ethyl bytyrate}}] \cdot 100\%$$
$$= 1/3 \cdot [\mathbf{Wt}_{\text{ethyl bytyrate}} / \mathbf{Wt}_{\text{tributyrin}}] \cdot [302.4/116.2] \cdot 100\%$$
$$= 0.867 \cdot [\mathbf{Wt}_{\text{ethyl butyrate}} / \mathbf{Wt}_{\text{tributyrin}}] \cdot 100\%$$

The following results were found:

EP 0 469 049 B1

| | Ex. 1 | Ex. 2 | Ex. 3 | Ex. 4 |
|---|---|---|---|---|
| %EPA | 10 | 29 | 38 | 55 |
| %DHA | 10 | 30 | 28 | 33 |
| % incorp.* 24 hrs | | | | |
| min. | 80 | 81 | 82 | 87 |
| max. | 83 | 85 | 86 | 91 |
| % incorp.* 48 hrs | | | | |
| min. | 90 | 86 | 90 | 91 |
| max. | 93 | 90 | 94 | 95 |
| % incorp.* 72hrs | | | | |
| min. | 92 | 92 | 94 | 93 |
| max. | 96 | 96 | 97 | 97 |

* The incorporation was determined by mass measurements of the entrapped product. The minimum incorporation was based on the assumption that all the moisture had escaped from the immobilized lipase.

These results show that essentially complete incorporation of fatty acids with a high content of PUFA, is possible with tributyrin, using stoichiometric amounts of the reactants.

## EXAMPLE 5

The preparation of triglycerides containing 99% EPA

Immobilized lipase SP 382 (0.65 g; 10% moisture-content) was added to a mixture of tributyrin (99% from Aldrich; 1.48 g, 4.89 mmol) and 99% EPA ethyl ester concentrate (M.wt. 330.5 g/mol; 4,87 g, 14.7 mmol). The mixture was gently stirred at 65°C on a magnetic stirrer hot-plate under a continous vacuum of 0.5 - 0.1 mmHg. The volatile ethyl butyrate product was continuously condensed into a liquid nitrogen cooled trap, which could be separated and weighed regularly during the process by disconnecting the reaction by replacing the vacuum with dry nitrogen or argon atmosphere. After 72 hours the reaction was discontinued, hexane added and the enzyme separated off by filtration. After solvent removal in vacuo on rotary evaporator the crude reaction product was afforded (4.43 g, 95%). Hydrolysis was found to occur only to a minimum extent as indicated by titration, which demonstrated less than 1% free fatty acid content of the product (0.7%) and further established by iatroscan studies. The weight measurements indicated 95-98% incorporation, iatroscan studies 97% and NMR spectorscopy 95%. The product was freed from the remaining ethyl esters and purified by the aid of prep. HPLC eluting with 10% ether in hexane to afford 100% pure triglycerides (3.80 g, 86% recovery equivalent to 82% overall yield) as established by iatroscan studies.

NMR spectroscopy indicated very pure material with only traces of the butyric acid moiety left in the triglycerides. [250 MHz $^1$H NMR (CDCl$_3$): $\delta$ 5.41-5.26 (m, 31 H, =C-H and -CH$_2$-CH-CH$_2$-), 4.30 (dd,J = 11.90 Hz, J = 4.34 Hz, 2 H, -CH$_2$-CH-CH$_2$-), 4.14 (dd,J = 11.90 Hz, J = 5.93 Hz, 2 H, -CH$_2$-CH-CH$_2$-), 2.90-2.78 (m, 24 H, =C-CH$_2$-C=), 2.33 (t,J = 7.34 Hz, 2H, OOC-CH$_2$-), 2.32 (t,J = 7.36 Hz, 4 H, OOC-CH$_2$-), 2.15-2.01 (m, 12 H, -CH$_2$-CH$_2$-C=), 1.75-1.61 (m, 6H, =CH-CH$_2$-CH$_3$), and 0.97 ppm (t,J = 7.52 Hz, 9 H, -CH$_3$). $^{13}$C NMR (CDCl$_3$): $\delta$ 172.9(s), 172.6(s), 132.0(d), 128,9(d), 128.7(d), 128.5(d), 128.2(d), 128.1(d), 128.1(d), 128.0(d), 127.8(d), 127.0(d), 68.9(d), 62.1(t), 33.5(t), 33.3(t), 26.4(t), 25.6(t), 25.6(t), 25.5 (t), 24.7(t), 24.7(t), 24.6(t), 20.5(t) and 14.2 ppm (q), IR (neat liquid): $v_{max}$ 3020 (vs,C=C-H), 2970 (S, CH$_3$), 2935 (s, CH$_2$), 2875 (s, CH$_3$), 2850 (w, CH$_2$), 1745 (vs, C=O) and 1645 cm$^{-1}$ (ms, C=C).]

The following results were obtained:

5

| Time | % incorporation | | |
|------|--------|--------|-----|
| hours | min.* | max.* | NMR |
| 2 | 53 | 57 | 64 |
| 4 | 67 | 70 | 73 |
| 8 | 80 | 83 | 82 |
| 24 | 90 | 93 | 91 |
| 48 | 94 | 97 | 93 |
| 72 | 95 | 98 | 95 |

* The incorporation was determined by mass measurements of the entrapped product. The minimum incorporation was based on the assumption that all the moisture had escaped from the immobilized lipase.

## EXAMPLE 6

The preparation of triglycerides containing 99% DHA

Immobilized lipase SP 382 (0.65 g; 10% moisture-content) was added to a mixture of tributyrin (99% from Aldrich; 1.38 g, 4.56 mmol) and 99% DHA ethyl ester concentrate (M.wt. 356.6 g/mol; 4,87 g, 13.6 mmol). The mixture was gently stirred at 65°C on a magnetic stirrer hot-plate under a continous vacuum of 0.5 - 0.1 mmHg. The volatile ethyl butyrate product was continuously condensed into a liquid nitrogen cooled trap, which could be separated and weighed regularly during the process by disconnecting the reaction by replacing the vacuum with dry nitrogen or argon atmosphere. After 72 hours the reaction was discontinued, hexane added and the enzyme separated off by filtration. After solvent removal in vacuo on rotary evaporator the crude reaction product was afforded (4.40 g, 94%). Hydrolysis was found to occur only to a minimum extent as indicated by titration, which demonstrated about 1% free fatty acid content of the product (1.2%) and further established by iatroscan studies. The weight measurements indicated 95-99% incorporation, iatroscan 96%, but NMR spectorscopy indicated 94%. The product was freed from the remaining ethyl esters and purified by the aid of prep. HPLC eluting with 10% ether in hexane to afford 100% pure triglycerides (3.56 g, 81% recovery equivalent to 76% overall yield) as established by iatroscan studies.

NMR spectroscopy indicated very pure material with only traces of the butyric acid moiety left in the triglycerides. [250 MHz $^1$H NMR (CDCl$_3$): $\delta$ 5.44-5.25 (m, 37 H, =C-H and -CH$_2$-CH-CH$_2$-), 4.30 (dd,J = 11.90 Hz, J = 4.36 Hz, 2 H, -CH$_2$-CH-CH$_2$-), 4.15 (dd,J = 11.90 Hz, J = 5.89 Hz, 2 H, - CH$_2$-CH-CH$_2$-), 2.90-2.79 (m, 30 H, =C-CH$_2$-C=), 2.39-2.38 (m, A$_2$B$_2$, 12 H, =CH-CH$_2$-CH$_2$-COOH), 2.13-2.02S (m, 6 H, =CH-CH$_2$- CH$_3$), and 0.97 ppm (t,J = 7.53 Hz, 9 H, -CH$_3$). $^{13}$C NMR (CDCl$_3$): $\delta$ 172.5(s), 172.1(s), 132.0(d), 129.5(d), 128.5(d), 128.3(d), 128.3(d), 128.2(d), 128.2(d), 128.0(d), 127.9(d), 127.8(d), 127.6(d), 127.0(d), 69.0(d), 62.2(t), 34.0(t), 33.8(t), 25.6(t), 25.6(t), 25.6(t), 25.6(t), 25.5(t), 22.6(t), 20.5(t) and 14.2 ppm(q), IR (neat liquid) : $v_{max}$ 3020 (vs,C=C-H), 2970 (s, CH$_3$), 2930 (s, CH$_2$), 2870 (s, CH$_3$), 2850 (w, CH$_2$), 1750 (vs, C=O) and 1650 cm$^{-1}$ (ms, C=C).]

The following results were obtained:

| Time | % incorporation | | |
|------|--------|--------|-----|
| hours | min.* | max.* | NMR |
| 2 | 32 | 35 | 33 |
| 4 | 63 | 67 | 63 |
| 8 | 77 | 81 | 78 |
| 24 | 91 | 95 | 89 |
| 48 | 94 | 98 | 94 |
| 72 | 95 | 99 | 94 |

*  The incorporation was determined by mass measurements of the
   entrapped product. The minimum incorporation was based on the
   assumption that all the moisture had escaped from the
   immobilized lipase.

## EXAMPLE 7

Preparation of triglycerides with reuse of lipase

Immobilized lipase (as in Ex. 1-4; 8.25g) was added to a mixture of tributyrin (20.0g; 66.1 mmol) and ethyl esters of cod liver oil (9% EPA and 9% DHA; M.wt. 313.1 g/mol; 62.1g; 199 mmol). The mixture was gently stirred at 65°C under a continuous vacuum of 0.1 mm Hg. The volatile ethyl butyrate product was condensed into a liquid nitrogen cooled trap, which was weighed regularly during the progress of the reaction. After 72 hours the reaction was discontinued and the lipase directly separated off without an organic solvent by filtration under dry nitrogen by the aid of a pressure equalized funnel equipped with a sintered glass filter plate and inlets/outlets to the nitrogen and the vacuum lines to aid the filtration, which was controlled by teflon key stopcocks. The oil was collected for further analysis.

The immobilized lipase was reintroduced into the reaction vessel. This was repeated 4 times, reusing the same immobilized lipase.

The following results were obtained:

| Run no. | % incorporation max.* |
|---------|----------------------|
| 1 | 93 |
| 2 | 86 |
| 3 | 85 |
| 4 | 87 |
| 5** | 89 |

*  As determined by mass measurements of the entrapped product
   after 72 hours.
** Results for the moisture-free lipase

7

**EXAMPLE 8**

Preparation of EPA/DHA triglyceride using triacetin

Example 1 was repeated, but with triacetin instead of tributyrin, and with 10% excess of ethyl esters. Water was observed to go off rapidly, so for calculation of incorporation, all water present was assumed to be included in the trapped weight. After 45 hours 72% incorporation had occurred, and after 69 hours 83%.

The results show that a high degree of incorporation can be achieved with triacetin, but water tends to go off (presumably due to formation of an azeotrope with ethyl acetate) , so stepwise addition of lipase may be advantageous to compensate for enzyme deactivation (due to water loss).

**Claims**

1. A process for the preparation of a triglyceride having at least one $C_{8+}$ long-chain fatty acid in the molecule, characterized by interesterification in the presence of a lipase of the long-chain free fatty acid or $C_1$-$C_4$ lower alkyl ester thereof with a triglyceride having one or more $C_2$-$C_6$ short-chain fatty acids in the molecule, and by evaporative removal during the reaction of short-chain free acid or lower alkyl ester thereof.

2. A process according to Claim 1 wherein the long-chain fatty acid is mono-, di- or polyunsaturated $C_{18-22}$.

3. A process according to any preceding claim, wherein the free acid or lower alkyl ester initially present comprises at least 90% of one or more long-chain acids or esters thereof.

4. A process according to any preceding claim for preparing a triglyceride with three long-chain acids the molecule.

5. A process according to any preceding claim, using a stoichiometric excess of 0-50% of the long chain fatty acid or ester relative to the short-chain fatty acid the triglyceride molecule.

6. A process according to Claim 5, wherein the reaction is continued until at least 90% by weight of the short-chain acid in the triglyceride is replaced.

7. A process according to any preceding claim, wherein the reactant triglyceride is tributyrin or triacetin.

8. A process according to any preceding claim, wherein the reactant free acid or ester is a free fatty acid or a methyl or ethyl ester.

9. A process according to any preceding claim, wherein the lipase is positionally non-specific.

10. A process according to any preceding claim, wherein the lipase is in immobilized form.

11. A process according to any preceding claim, wherein the removal of acid ester is done under vacuum.

12. A triglyceride composition, characterized by at least 95 % by weight of the fatty acids in the triglyceride molecules being polyunsaturated $C_{18-22}$ acid.

13. A composition according to Claim 12, wherein the polyunsaturated acid is eicosapentaenoic acid, docosahexaenoic acid or a mixture of these.

**Patentansprüche**

1. Verfahren zur Herstellung eines Triglycerids mit wenigstens einer langkettigen $C_{8+}$-Fettsäure im Molekül, gekennzeichnet durch die Umesterung der (des) langkettigen freien Fettsäure oder $C_1$-$C_4$-Niederalkylesters derselben mit einem Triglycerid mit einer oder mehreren kurzkettigen $C_2$-$C_6$-Fettsäuren im Molekül in Gegenwart einer Lipase und Entfernen von kurzkettiger(m) freier Säure oder Niederalkylester derselben durch Verdampfung während der Reaktion.

8

2. Verfahren nach Anspruch 1, dadurch gekennzeichnet, daß die langkettige Fettsäure einfach, zweifach oder mehrfach ungesättigte $C_{18-22}$ ist.

3. Verfahren nach irgendeinem vorangehenden Anspruch, dadurch gekennzeichnet, daß die anfänglich vorliegende freie Säure oder der anfänglich vorliegende Niederalkylester wenigstens 90 % an einer oder mehreren langkettigen Säuren oder Estern derselben umfaßt.

4. Verfahren nach irgendeinem vorangehenden Anspruch zur Herstellung eines Triglycerids mit drei langkettigen Säuren im Molekül.

5. Verfahren nach irgendeinem vorangehenden Anspruch, dadurch gekennzeichnet, daß ein stöchiometrischer Überschuß von 0-50 % der (des) langkettigen Fettsäure oder Esters relativ zur kurzkettigen Fettsäure im Triglycerid-Molekül eingesetzt wird.

6. Verfahren nach Anspruch 5, dadurch gekennzeichnet, daß die Reaktion fortgesetzt wird, bis wenigstens 90 Gew.-% der kurzkettigen Säure im Triglycerid ersetzt sind.

7. Verfahren nach irgendeinem vorangehenden Anspruch, dadurch gekennzeichnet, daß der Reaktant Triglycerid Tributyrin oder Triacetin ist.

8. Verfahren nach irgendeinem vorangehendem Anspruch, dadurch gekennzeichnet, daß der Reaktant freie Säure oder der reagierende Ester eine freie Fettsäure oder ein Methyl-oder Ethylester ist.

9. Verfahren nach irgendeinem vorangehenden Anspruch, dadurch gekennzeichnet, daß die Lipase nicht positionsspezifisch ist.

10. Verfahren nach irgendeinem vorangehenden Anspruch, dadurch gekennzeichnet, daß die Lipase in immobilisierter Form vorliegt.

11. Verfahren nach einem vorangehenden Anspruch, dadurch gekennzeichnet, daß das Entfernen von Säure oder Ester unter Vakuum durchgeführt wird.

12. Triglyceridzusammensetzung, dadurch gekennzeichnet, daß wenigstens 95 Gew.-% der Fettsäuren in den Triglycerid-Molekülen mehrfach ungesättigte $C_{18-22}$-Säure sind.

13. Zusammensetzung nach Anspruch 12, dadurch gekennzeichnet, daß die mehrfach ungesättigte Säure Eicosapentaensäure, Docosahexaensäure oder eine Mischung aus diesen ist.

## Revendications

1. Procédé pour la préparation d'un triglycéride ayant au moins un acide gras à chaîne longue en $C_{8+}$ dans la molécule, caractérisé par l'interestérification en présence d'une lipase de l'acide gras libre à chaîne longue ou de son ester d'alkyle inférieur en $C_1$-$C_4$ avec un triglycéride ayant un ou plusieurs acides gras à chaîne courte en $C_2$-$C_6$ dans la molécule et par extraction par évaporation au cours de la réaction de l'acide libre à chaîne courte ou de son ester d'alkyle inférieur.

2. Procédé selon la revendication 1, dans lequel l'acide gras à chaîne longue est mono-, di- ou polyinsaturé en $C_{18-22}$.

3. Procédé selon l'une quelconque des revendications précédentes, dans lequel l'acide libre ou l'ester d'alkyle inférieur présent à l'origine comprend au moins 90 % d'un ou plusieurs acides à chaîne longue ou ses esters.

4. Procédé selon l'une quelconque des revendications précédentes, pour la préparation d'un triglycéride avec trois acides à chaîne longue dans la molécule.

5. Procédé selon l'une quelconque des revendications précédentes, en utilisant l'excédent stoéchiométrique de 0-50 % de l'acide gras à chaîne longue ou de l'ester par rapport à l'acide gras à chaîne courte dans la molécule de triglycéride.

6. Procédé selon la revendication 5, dans lequel la réaction est poursuivie jusqu'au remplacement d'au moins 90% en poids de l'acide à chaîne longue dans le triglycéride.

7. Procédé selon l'une quelconque des revendications précédentes, dans lequel le triglycéride réactif est la tributyrine ou la triacétine.

8. Procédé selon l'une quelconque des revendications précédentes, dans lequel l'acide libre réactif ou l'ester est un acide gras libre ou un ester de méthyle ou d'éthyle.

9. Procédé selon l'une quelconque des revendications précédentes, dans lequel la lipase est non spécifique du point de vue position.

10. Procédé selon l'une quelconque des revendications précédentes, dans lequel la lipase est dans la forme immobilisée.

11. Procédé selon l'une quelconque des revendications précédentes, dans lequel l'extraction de l'ester d'acide s'effectue sous vide.

12. Composition de triglycéride, caractérisée en ce qu'au moins 95 % en poids des acides gras dans les molécules de triglycéride sont constitués par l'acide polyinsaturé en $C_{18-22}$.

13. Composition selon la revendication 12, dans laquelle l'acide polyinsaturé est l'acide eicosapentaénoïque, l'acide docosahexaénoïque ou un mélange de ceux-ci.